# EUROPEAN PATENT SPECIFICATION

(11) **EP 3 063 542 B1**
(45) Date of publication and mention of the grant of the patent: **20.01.2021**
(21) Application number: 14809984.9
(22) Date of filing: 31.10.2014
(51) Int. Cl.: G01N 33/571, G01N 33/68

(54) **METHOD FOR DIAGNOSING AN INFLAMMATORY CONDITION IN THE FEMALE GENITAL TRACT**
VERFAHREN ZUR DIAGNOSE EINER ENTZÜNDUNGSKRANKHEIT IM WEIBLICHEN GENITALTRAKT
MÉTHODE DE DIAGNOSTIC D'UNE AFFECTION INFLAMMATOIRE TOUCHANT L'APPAREIL GÉNITAL FÉMININ

(30) Priority: 31.10.2013 GB 201319264
(43) Date of publication of application: 07.09.2016
(73) Proprietor: University of Cape Town, Cape Town 7700 (ZA)
(72) Inventor: MASSON, Lindi, 7945 Cape Town (ZA); PASSMORE, Jo-Ann Shelley, 7945 Cape Town (ZA)
(74) Representative: Potter Clarkson
(86) International application number: PCT/IB2014/065740
(87) International publication number: WO 2015/063730

(56) References cited:
- US-A1- 2009 159 812
- Elizabeth St. John ET AL: "Bacterial vaginosis and host immunity", Current HIV/AIDS Reports, 1 February 2007 (2007-02-01), pages 22-28, XP055164092, New York DOI: 10.1007/s11904-007-0004-y Retrieved from the Internet: URL:http://www.ncbi.nlm.nih.gov/pubmed/173 38857
- RYCKMAN K K ET AL: "Racial differences in cervical cytokine concentrations between pregnant women with and without bacterial vaginosis", JOURNAL OF REPRODUCTIVE IMMUNOLOGY, ELSEVIER SCIENCE IRELAND LTD, IE, vol. 78, no. 2, 1 July 2008 (2008-07-01), pages 166-171, XP022734116, ISSN: 0165-0378, DOI: 10.1016/J.JRI.2008.01.003 [retrieved on 2008-03-11]
- GREGORY T. SPEAR ET AL: "Multiplex Immunoassay of Lower Genital Tract Mucosal Fluid from Women Attending an Urban STD Clinic Shows Broadly Increased IL1ß and Lactoferrin", PLOS ONE, vol. 6, no. 5, 10 May 2011 (2011-05-10), page e19560, XP055163916, DOI: 10.1371/journal.pone.0019560

## Description

### FIELD OF THE INVENTION

The invention relates to the use of cytokines measured in the female genital tract as biomarkers of inflammatory conditions, in particular sexually transmitted infections (STIs) and bacterial vaginosis (BV).

### BACKGROUND OF THE INVENTION

Sexually transmitted infections (STIs) and bacterial vaginosis (BV) cause inflammation in the female genital tract that is associated with increased risk of HIV infection.

Nucleic acid amplification tests (NAATs) for specific STIs are the gold standard for STI diagnosis. However, compared to syndromic management, these tests are costly and they require experienced laboratory personnel and equipment, which is often lacking in resource-limited settings (WHO, 2003). Onward transmission of STIs may occur while individuals await their results and many individuals don't return to the clinics for their results and are thus left untreated.

Rapid point-of-care (POC) tests have been under development for several years and have been successfully used for HIV and syphilis diagnosis. However, most rapid tests for STIs show differing predictive values in different studies. While some studies have reported that the Chlamydia Rapid Test (CRT) has shown promise, with a sensitivity of 80% and specificity of 99% (Hislop et al., 2010), others demonstrated that CRT had a sensitivity of only 41% (van der Helm et al., 2012). Neisseria gonorrhoeae leukocyte esterase tests have shown sensitivities ranging between only 23 and 86% and immunochromatographic strip tests have shown sensitivities between 60 and 70% in different studies (Smith et al., 2012). A Trichomonas vaginalis rapid antigen test has been reported to be 90-95% sensitive and 100% specific (Huppert et al., 2007; Campbell et al., 2008). Rapid combination NAATs (which give results in <2 hrs) for chlamydia and gonorrhoea have been developed and show promising predictive value (chlamydia: sensitivity 98.7%; specificity 99.4%; gonorrhoea: sensitivity 100%; specificity 99.8%). However, these tests require an onsite GeneXpert Platform and may be too expensive to be implemented in resource limited settings. Recently, a highly sensitive and specific rapid NAAT test for T. vaginalis was also described (which gives results in 30 minutes). However, an onsite PCR machine is also required for this test (Pearce et al., 2013).

In many developing countries, BV and STIs are therefore managed according to signs and symptoms, because this approach is easily implemented, relatively inexpensive and patients are given immediate treatment.

However, large proportions of women who have BVs or STIs are asymptomatic and are thus left untreated (Wilkinson et al., 1999; Mlisana et al., 2012). Women with asymptomatic STIs have comparable levels of inflammation in their genital tracts to women with symptomatic infections, which are elevated compared to women who do not have an STI or BV (Mlisana et al., 2012). Many women are thus likely to have STI-related inflammation that remains unresolved, placing them at increased risk of HIV infection, as well as reproductive complications (Linder et al., 1990; McNeely, 1992; Heine and McGregor, 1993; Cotch et al., 1997; Moodley and Sturm, 2000; Mlisana et al., 2012). These findings highlight the need for better STI management strategies in developing countries.

There is thus an urgent need for a new, accurate and inexpensive POC test to identify women with asymptomatic STIs/BV who have subclinical inflammation and are at increased risk of HIV infection.

St. John, E., et al., Current HIV/AIDS Reports, 22-28, (2007) describes the correlation between BV and higher susceptibility to infection of HIV and other genital tract pathogens.

Ryckman, K. K., et al., J. Reprod. Immunol., 78(2), 166-171, (2008) describes differing levels of IP-10 and IL-1 in pregnant women with and without BV.

Spear, G. T., et al., PLoS ONE, 6(5), (2011) describes the levels of IP010 and IL-1 beta measured in women with sexually transmitted diseases.

### SUMMARY OF THE INVENTION

According to a first embodiment of the invention, there is provided a method of non-specifically diagnosing asymptomatic bacterial vaginosis (BV) and an asymptomatic sexually transmitted infection (STI) in the genital tract of a non-pregnant female subject in a single test, the method comprising the steps of:
a) measuring the levels of IP-10 and either of IL-1β or IL-1α in a sample from the subject;
b) comparing each of these levels to a pre-determined level of IP-10 and either of IL-1β or IL-1α, and
c) if the level of IP-10 in the sample is lower than the predetermined level of IP-10 or if the level of IL-1β or IL-1α in the sample is higher than the predetermined level of IL-1β or IL-1α, then making a non-specific diagnosis that the subject has at least one of asymptomatic bacterial vaginosis and an asymptomatic sexually transmitted infection.

If the subject is diagnosed as having an inflammatory condition, which may be either BV or an STI, she may be provided with medication which is capable of treating both BV and an STI or may be referred for further testing.

The method may be performed in the absence of clinical symptoms of inflammation in the genital tract, BV or an STI.

The sample may comprise cervicovaginal fluid from the subject, such as from cervicovaginal lavage, a vulvovaginal swab, a lateral wall swab, a cervical softcup or the like.

The levels or concentrations of IP-10 and either of IL-1β or IL-1α may be measured in the sample without any stimulation thereof.

The method may be performed on an HIV-infected or HIV-uninfected subject.

A test kit for non-specifically diagnosing an inflammatory condition in the genital tract of a non-pregnant female subject according to the method described above, the kit comprising:
(a) detecting means for detecting the levels of IP-10 and either of IL-1β or IL-1α in a sample from the subject; and
(b) indicating means for indicating that the subject has an inflammatory condition in the genital tract caused by either bacterial vaginosis or a sexually transmitted infection,
is also described.

### BRIEF DESCRIPTION OF THE DRAWINGS

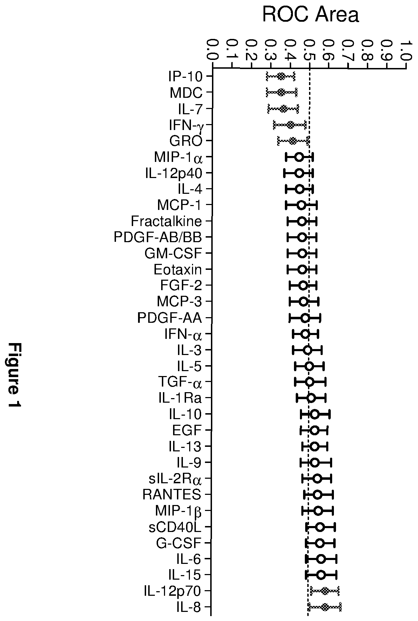

- **Figure 1**: shows Receiver Operating Characteristic (ROC) areas for each of the 42 cytokines assessed for this invention. Cytokines with a ROC area of 0.5 have no predictive value. Cytokines with a ROC area >0.5 are positively associated with the presence of an STI or BV, whereas cytokines with a ROC area <0.5 are inversely associated. The first 5 and last 10 cytokines were found to be significantly associated with the presence of an STI of BV.

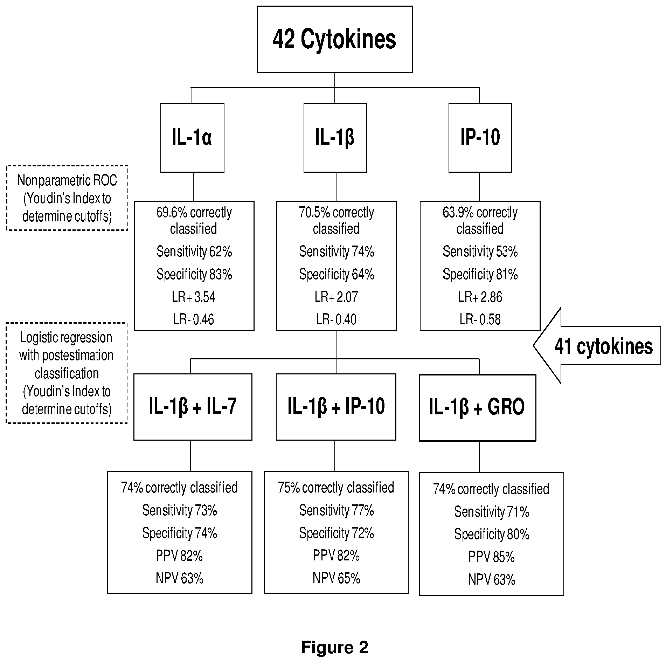

- **Figure 2**: shows a flow diagram identifying potential cytokine biomarkers of STIs or BV. The predictive value of each of the 42 cytokines measured in this study was assessed using Receiver Operating Characteristic (ROC) curves. Youdin's Index was used to determine appropriate cutoffs for each cytokine. IL-1β was found to be best predictive of the presence of an STI or BV and was therefore used as a starting point to build a logistic regression model that included the cytokines that together most strongly predicted the presence of an STI or BV.

### DETAILED DESCRIPTION OF THE INVENTION

The present invention provides a method as defined in Claim 1. The method measures the concentration of IP-10 (interferon-γ induced protein (IP)-10) and either of IL-1β (interleukin (IL)-1β) or IL-1α (interleukin (IL)-1α) in a sample of cervicovaginal fluid from the subject and compares these to predetermined levels of IP-10 and either of IL-1β or IL-1α, respectively. A down-regulated IP-10 level in the sample or an up-regulated IL-1β or IL-1α level in the sample will be regarded as a positive result, i.e. indicating that there is a high probability that the subject has an inflammatory condition, which may be either bacterial vaginosis (BV) or a sexually transmitted infection (STI). The subject could then either be directly treated, e.g. with broad spectrum antibiotics which are capable of treating both BV and STIs, or referred for appropriate STI and BV screening.

The method may additionally measure the levels of other cytokines, such as TNF-α, TNF-β, MDC, IL-7, IFN-γ and GRO.

Genital cytokine changes associated with bacterial vaginosis in pregnant women have previously been studied to better understand immunological predictors of preterm delivery (Ryckman et al., Journal of Reproductive Immunology, vol. 78, 2008; Platz-Christensen et al., American Journal of Obstetrics and Gynecology, vol. 169, 1993; Mattsby-Baltzer et al., Acta Obstet. Gynecol. Scand., vol. 77, 1978). However, the results from these studies were not consistent. Studies have also shown that genital cytokine profiles change during pregnancy (Kutteh and Franklin, 2001; Donders et al., 2003; Raghupathy and Kalinka, 2008) and cytokine responses to bacterial vaginosis also differ between pregnant and non-pregnant women (Beigi et al., 2007). Of particular relevance to this application, IL-1β concentrations were higher in the genital tracts of pregnant women with bacterial vaginosis compared to non-pregnant women with bacterial vaginosis (Beigi et al., 2007). Further, genital IL-1β levels have been shown to increase significantly from the first trimester to the second trimester (Kutteh and Franklin, 2001). Therefore cytokine biomarkers that are predictive of bacterial vaginosis in pregnant women are not likely to be applicable to non-pregnant women.

The method of the invention enables the identification of women who have asymptomatic STIs or BV in a single test. A broad (non-specific) test for the presence of any STI (including *Chlamydia trachomatis, N. gonorrhoeae, T. vaginalis, M. genitalium*) or BV will be useful, as women with these infections have the same phenotype of inflammation, irrespective of the specific STI/BV, and common STIs/BV are treatable with the same broad spectrum antibiotics. Also, it would be much more efficient and cost-effective to perform only one test instead of multiple tests for each infectious agent.

It is anticipated that identification and treatment of women with asymptomatic STIs/BV could reduce the prevalence of STIs and BV. Because STIs and BV and associated inflammation are predictors of HIV risk, better management of STIs and BV using this approach may also reduce incidence of HIV transmission. In regions of high HIV prevalence and high STI burden, such as South Africa, this could have considerable economic and societal benefits.

The sample can be obtained from a vaginal secretion from the subject, such as from a vulvovaginal swab or cervicovaginal lavage.

Unlike other diagnostic methods for STIs or BV, the cytokine levels which are measured are those which are found in the patient, and the sample does not have to be stimulated with an antigen.

The predetermined levels of IP-10, IL-1β and/or IL-1α will be based on comparisons of levels of these cytokines observed in women infected with STIs/BV and women with normal flora or uninfected women. A level of IP-10 will be selected below which it is likely that the woman has BV. A level of IL-1β and/or IL-1α will be selected above which it is likely that the woman has an STI or BV.

As described in more detail below, the concentrations of 42 cytokines were measured in cervicovaginal lavage (CVL) by Luminex from 227 HIV-uninfected and 22 cytokines in CVL from 38 HIV-infected women from Durban, South Africa, who were screened for several common STIs and BV. In order to compare different methods of cytokine measurement, Cytometric Bead Array (CBA) was used to measure the concentrations of 10 cytokines in CVL from 222/227 of the HIV-uninfected women.

It was surprisingly found that a model including upregulated IL-1β and downregulated IP-10 was best predictive of the presence of an STI or BV in the cohort of HIV-uninfected women [with a sensitivity of 77%, specificity 72%, positive predictive value (PPV) 82% and negative predictive value (NPV) 65%]. This model had substantially improved sensitivity compared to clinical signs (19%), detecting 58% more women who had an STI or BV. The predictive value of these biomarkers was found to be similar when measured using CBA. Upon resampling of ten 3/4 subsets of the original HIV-uninfected cohort and reapplication of the model, it was found that the predictive value of IL-1β and IP-10 remained consistent. Additionally, in HIV-infected women, the predictive value of IL-1β and IP-10 was even better than observed for HIV negative women (sensitivity 80%, specificity 100%, PPV 100%, NPV 54%). These findings demonstrate that IL-1β and IP-10 may be useful as biomarkers in a rapid point of care test aimed at identifying women with asymptomatic STIs or BV.

The results for IL-1α were also promising, and it is envisaged that this cytokine could be measured in the method of the invention instead of (or in addition to) IL-1β.

Additionally, TNF-α, TNF-β, MDC, IL-7, IFN-γ and GRO were found to have predictive potential and any combinations of these cytokines could serve as useful additional biomarkers to identify women with STIs and/or BV. Alternatively, using this test in combination with another, existing rapid POC test might help to improve the predictive value.

Further research will be conducted to develop a POC test based upon the method described above, the test being designed to give rapid results, to be inexpensive and easy to use, and not require sophisticated equipment. For example, a test kit could include one or more of receiving means for receiving a sample from the subject; detecting means for detecting the levels of IP-10 and either of IL-1β or IL-1α in the sample; indicating means for indicating that the subject has a genital inflammatory condition; instructions for using the kit; and so forth. A person skilled in the art will be aware that there are various assay systems available for quantifying concentrations of cytokines and which could be used in the test. For example, the detection of the biomarkers may form part of an integrated microfluidic device or a lateral flow assay.

The invention will now be described in more detail with reference to the following non-limiting examples.

### Examples

### Study participants

The participants of this study included 227 high-risk HIV-uninfected and 38 recently HIV-infected women enrolled in the Centre for the AIDS Programme of Research in South Africa (CAPRISA) 002 study. All women provided informed consent and this study was approved by the University of KwaZulu-Natal and University of Cape Town Ethics Committees.

### STI and BV testing

Each woman was screened for STIs and BV as previously described (Mlisana et al., 2012). Briefly, a gynaecological examination was performed and two vulvovaginal swabs were collected from the anterior and posterior fornices and lateral vaginal walls. All specimens were transported to the diagnostic laboratory (Medical Microbiology Laboratory, University of KwaZulu-Natal) within one hour of collection for same day processing. *Chlamydia trachomatis, Neisseria gonorrhoeae, Mycoplasma genitaliumm Trichomonas vaginalis* and herpes simplex virus (HSV) were assessed by PCR. *N. gonorrhoea* and *C. trachomatis* were isolated using the BDProbe Tec™ ET assay (Becton Dickinson Microbiology systems, U.S.A.). One swab was rolled onto a glass slide for Gram staining for diagnosis of BV using Nugents criteria. Blood specimens were collected for syphilis (*Treponema pallidum*) diagnosis and HSV-2 serology. ELISA was used to detect IgG antibodies to HSV-2 gG-2 antigen (HerpeSelect®, Focus Diagnositics, CA, USA). Syphilis screening was done using the Becton Dickinson Macro-VueTM Rapid Plasma Reagin (RPR) card test followed by a haemagglutination test (ImmuTrep® TPHA, Omega Diagnostics LTD, Scotland, UK) to identify antibodies against *T. pallidum.*

### Cytokine measurements

Cervicovaginal lavage (CVL) samples for cytokine measurements were collected as follows: Sterile normal saline (10ml) was used to repeatedly bathe the cervix and allowed to pool in the posterior fornix, where it was then aspirated into a plastic bulb pipette (Bebell *et al.,* 2008). Samples were centrifuged and supernatant stored at -80 °C. CVL samples were not collected from menstruating participants. After thawing, CVLs were pre-filtered by centrifugation using 0.2 µm cellulose acetate filters (Sigma, U.S.A.). The concentrations of 42 cytokines were measured in CVL samples using Human Cytokine LINCO*plex* Premixed kits (LINCO Research, MO, USA). Cytokines measured included epidermal growth factor (EGF), eotaxin/CCL11, FGF-2, fms-like tyrosine kinase-3 Ligand (FLT3L), fractalkine/CX3CL1, granulocyte colony stimulating factor (G-CSF), granulocyte macrophage (GM)-CSF, growth related oncogene (GRO) family (CXCL1-CXCL3), interferon (IFN)-α, IFN-γ, interleukin (IL)-1α, IL-1β, IL-1Ra, IL-2, IL-3, IL-4, IL-5, IL-6, IL-7, IL-8/CXCL8, IL-9, IL-10, IL-12p40, IL-12p70, IL-13, IL-15, IL-17, IFN-γ-induced protein 10 (IP-10)/CXCL10, monocyte chemotactic protein (MCP-1)/CCL2, MCP-3/CCL7, macrophage-derived chemokine (MDC)/CCL22, macrophage inflammatory protein (MIP-1α)/CCL3, MIP-1β/CCL4, platelet-derived growth factor (PDGF)-AA, PDGF-AB/BB, regulated upon activation normal T cell expressed and secreted (RANTES)/CCL5, soluble CD40 ligand (sCD40L), soluble IL-2 receptor α (sIL-2Rα), transforming growth factor (TGF)-α, tumour necrosis factor (TNF)-α, TNF-β, and vascular endothelial growth factor (VEGF). The lower limit of detection of these kits ranged between 0.01 and 27.65 pg/ml for each of the cytokines measured. Data was collected using a Bio-Plex™ Suspension Array Reader (Bio-Rad Laboratories Inc®) and a 5 PL regression formula was used to calculate cytokine concentrations from the standard curves. Data was analysed using BIO-plex manager software (version 4; Bio-Rad Laboratories Inc®). The concentrations of IL-1β, IL-6, IL-8, IL-10, IL-12p70, IP-10, MCP-1, MIG, TNF-α and RANTES were additionally measured in CVLs from 222/227 of the study participants using Human Inflammation and Chemokine Cytometric Bead Array (CBA) kits (BD Biosciences, Pharmingen, San Diego, CA) according to the manufacturer's instructions. Cytokine concentrations that were below the lower limit of detection of the assay were reported as the mid-point between the lowest concentration measured for each cytokine and zero.

### Statistical analysis

Statistical analyses were performed using STATA™ version 11. Cytokine data was log₁₀-transformed. Receiver operating characteristic (ROC) curves were used to compare the predictive values of each cytokine. Youdin's Index was used to determine appropriate cutoffs for each cytokine, assuming that sensitivity and specificity are of equal importance. Logistic regression was used to determine the variables that were together best predictive of the presence of an STI or BV. Variables that were significantly associated with the presence of an STI or BV were added to the models in a stepwise manner.

### Results

The median age of the HIV-uninfected women in this study was 36 years (range 18 - 58), while the HIV-infected women were a median of 25 years old (range 18 - 59). Fifty-three percent (120/227) of HIV-uninfected women had BV and 29% (68/227) were PCR positive for one or more active STI (*C*. *trachomatis, N. gonorrhoeae, M. genitalium, T. vaginalis* and/or HSV) or had a *T. pallidum* RPR titre >1:4. Seventy-four percent (28/38) of the HIV-infected women had BV and 40% (15/38) had an active STI (Table 1). Despite this, the prevalence of clinical signs of BV and STIs was low in both groups, with 34 HIV-uninfected women (15%) and 7 HIV-infected women (18%) having visible cervicovaginal discharge. Although 198 HIV-uninfected women (87%) and 36 HIV-infected women (92%) tested positive for HSV-2 IgG antibodies, only 6 HIV-uninfected and 2 HIV-infected women had detectable HSV in their genital tracts. None of the HIV-uninfected women and 4 of the HIV-infected women had visible genital ulceration.

**Table 1: Prevalence of BV and STIs**

| **Sexually transmitted infections** | **HIV-uninfected n/total (%)** | **HIV-infected n/total (%)** |
|---|---|---|
| Prevalence of active STIs (women with lab diagnosed STI) | 66/227 (29.1) | 15/38 (39.5) |
| Bacterial vaginosis (women gram stain positive for BV) | 120/227 (52.9) | 28/38 (73.7) |
| Cervicovaginal discharge | 34/227 (15.0) | 7/38 (18.4) |
| Genital ulceration | 0/227 (0.0) | 4/38 (10.5) |

The ability of each of the 42 cytokines measured in this study using Luminex to predict the presence of an STI/BV was assessed in HIV-uninfected women. It was found that IL-1α and IL-1β had the largest ROC areas, indicating that these cytokines have the highest sensitivities and specificities. Of the cytokines that were inversely associated with the presence of an STI or BV, IP-10 was best predictive of the presence of an STI or BV (Figure 1).

IL-1α, IL-1β and IP-10 were therefore selected for further analysis. Optimal cutoffs were determined using Youdin's Index, assuming that sensitivity and specificity are of equal importance. It was found that IL-1β had marginally better predictive value compared to IL-1α and IP-10 (Figure 2). Therefore, IL-1β was used as a starting point to develop a multivariate logistic regression model that included the cytokines that together most strongly predicted the presence of an STI or BV. Each of the other 41 cytokines was then stepwise added to IL-1β.

It was found that a model including IL-1β and IP-10 was best predictive of the presence of an STI/BV in this cohort of HIV-uninfected women (Figure 1), with a sensitivity of 77%, specificity of 72%, positive predictive value (PPV) of 82% and negative predictive value (NPV) of 65% (Table 2). Inclusion of a third cytokine did not improve the predictive value of the model. Upon evaluation of model performance by reapplication of the model to 10 randomly-chosen three-quarter subsets of the study group, the relationships between each cytokine and the presence of an STI/BV remained statistically significant and the directionality of the relationships between the cytokines and STIs/BV remained constant, indicating that the model estimates are stable. When applied to each subset of women, the model correctly classified 72 to 80% of women, demonstrating that the biomarkers had similar predictive value in each resampled subset of women.

The reproducibility of IL-1β and IP-10 measurements was evaluated by assessing the concentrations of these cytokines in CVLs from a subset of 222/227 women using a different method of cytokine measurement, CBA (Table 2). It was found that the predictive value of IL-1β and IP-10 was similar when these cytokines were measured using CBA (sensitivity 83%, specificity 61%, PPV 78%, NPV 69%; Table 2), compared to Luminex.

In order to determine whether IL-1β and IP-10 would also have potential as biomarkers of STIs and BV in HIV-infected women, their predictive value was investigated in a cohort of women who had recently acquired HIV infection (median 6 week post-infection). IL-1β and IP-10 were found to predict STIs and BV with similar sensitivity in HIV-infected women (80%), but improved specificity (100%), compared to HIV-uninfected women (Table 2).

**Table 2: Genital cytokine concentrations as biomarkers of a genital inflammatory condition caused by STIs and BV in HIV-uninfected and infected women**

| **Participants** | **Biomarker** | **Cytokine measurement** | **Classification** | **STI*/BV diagnosis (n)** | | **Sensitivity (%)** | **Specificity (%)** | **PPV (%)** | **NPV (%)** |
|---|---|---|---|---|---|---|---|---|---|
| | | | | **Pos** | **Neg** | | | | |
| HIV- | IL-1 β + IP-10 | Luminex | Pos | 108 | 24 | 77 | 72 | 82 | 65 |
| | | | Neg | 33 | 62 | | | | |
| HIV- | IL-1 β + IP-10 | CBA | Pos | 115 | 33 | 83 | 61 | 78 | 69 |
| | | | Neg | 23 | 51 | | | | |
| HIV+ | IL-1 β + IP-10 | Luminex | Pos | 25 | 0 | 80 | 100 | 100 | 54 |
| | | | Neg | 5 | 7 | | | | |
| HIV- | Discharge or ulceration | N/A | Pos | 27 | 7 | 19 | 92 | 79 | 40 |
| | | | Neg | 115 | 78 | | | | |
| HIV+ | Discharge or ulceration | N/A | Pos | 13 | 0 | 35 | 100 | 100 | 23 |
| | | | Neg | 24 | 7 | | | | |

| | | | | | | | | | |
|---|---|---|---|---|---|---|---|---|---|
| **Chlamydia trachomatis, Neisseria gonorrhoeae, Mycoplasma genitalium, Trichomonas vaginalis* and herpes simplex virus (HSV) were assessed by PCR. CBA: Cytometric Bead Array; PPV: Positive predictive value; NPV: Negative predictive value. | | | | | | | | | |

## Claims

1. A method of non-specifically diagnosing asymptomatic bacterial vaginosis and an asymptomatic sexually transmitted infection in the genital tract of a non-pregnant female subject in a single test, the method comprising the steps of:
a) measuring the levels of IP-10 and either of IL-1β or IL-1α in a sample from the subject;
b) comparing each of these levels to a pre-determined level of IP-10 and either of IL-1β or IL-1α, and
c) if the level of IP-10 in the sample is lower than the predetermined level of IP-10 or if the level of IL-1β or IL-1α in the sample is higher than the predetermined level of IL-1β or IL-1α, then making a non-specific diagnosis that the subject has at least one of asymptomatic bacterial vaginosis and an asymptomatic sexually transmitted infection.

2. A method according to claim 1, which includes the step of referring the subject for further testing to determine the particular condition or infection.

3. A method according to either of claims 1 or 2, wherein the diagnosis is made in the absence of clinical symptoms of inflammation in the genital tract, bacterial vaginosis or a sexually transmitted infection.

4. A method according to any one of claims 1 to 3, wherein the sample comprises vaginal fluid which has been obtained from the subject.

5. A method according to claim 4, wherein the sample is from cervicovaginal lavage, a vulvovaginal swab, a lateral wall swab or a cervical softcup of the subject.

6. A method according to any one of claims 1 to 5, wherein step (a) is performed on an unstimulated sample.

7. A method according to any one of claims 1 to 6, wherein the levels of one or more additional cytokines selected from the group consisting of TNF-α, TNF-β, MDC, IL-7, IFN-γ and GRO are measured and compared to predetermined levels in order to make the diagnosis.

8. A method according to any one of claims 1 to 7, wherein the subject is HIV-positive.

9. A method according to any one of claims 1 to 7, wherein the subject is HIV-negative.

## Patentansprüche

1. Verfahren zum unspezifischen Diagnostizieren einer asymptomatischen bakteriellen Vaginose und einer asymptomatischen sexuell übertragbaren Infektion im Genitaltrakt eines nicht-schwangeren weiblichen Subjekts in einem einzigen Test, wobei das Verfahren die folgenden Schritte umfasst:
a) Messen der Werte von IP-10 und entweder von IL-1β oder IL-1α in einer Probe von dem Subjekt;
b) Vergleichen jedes dieser Werte mit einem zuvor bestimmten Wert von IP-10 und entweder IL-1β oder IL-1α, und
c) wenn der Wert von IP-10 in der Probe niedriger als der zuvor bestimmte Wert von IP-10 ist oder wenn der Wert von IL-1β oder IL-1α in der Probe höher als der zuvor bestimmte Wert von IL-1β oder IL-1α ist, dann Stellen einer unspezifischen Diagnose, dass das Subjekt eine asymptomatische bakterielle Vaginose und/oder eine asymptomatische sexuell übertragbare Infektion aufweist.

2. Verfahren nach Anspruch 1, das den Schritt eines Überweisens des Subjekts zum weiteren Untersuchen beinhaltet, um den speziellen Krankheitszustand oder die Infektion zu bestimmen.

3. Verfahren nach Anspruch 1 oder 2, wobei die Diagnose in der Abwesenheit von klinischen Symptomen einer Entzündung im Genitaltrakt, einer bakteriellen Vaginose oder einer sexuell übertragbaren Infektion gestellt wird.

4. Verfahren nach einem der Ansprüche 1 bis 3, wobei die Probe Vaginalflüssigkeit umfasst, die von dem Subjekt erhalten wurde.

5. Verfahren nach Anspruch 4, wobei die Probe aus einer zervikovaginalen Lavage, einem vulvovaginalen Abstrich, einem Seitenwandabstrich oder einem zervikalen Softcup des Subjekts stammt.

6. Verfahren nach einem der Ansprüche 1 bis 5, wobei Schritt (a) an einer unstimulierten Probe durchgeführt wird.

7. Verfahren nach einem der Ansprüche 1 bis 6, wobei die Werte eines oder mehrerer zusätzlicher Zytokine, die aus der Gruppe ausgewählt sind, die aus TNF-α, TNF-β, MDC, IL-7, IFN-γ und GRO besteht, gemessen und mit zuvor bestimmten Werten verglichen werden, um die Diagnose zu stellen.

8. Verfahren nach einem der Ansprüche 1 bis 7, wobei das Subjekt HIV-positiv ist.

9. Verfahren nach einem der Ansprüche 1 bis 7, wobei das Subjekt HIV-negativ ist.

## Revendications

1. Procédé de diagnostic non spécifique de la vaginose bactérienne asymptomatique et d'une infection sexuellement transmissible asymptomatique dans le tractus génital d'une patiente non gravide en un seul test, le procédé comprenant les étapes consistant à :
a) mesurer les niveaux d'IP-10 et d'IL-1β ou d'IL-1α dans un échantillon provenant de la patiente ;
b) comparer chacun de ces niveaux à un niveau prédéterminé d'IP-10 et soit d'IL-1β soit d'IL-1α et
c) si le niveau d'IP-10 dans l'échantillon est inférieur au niveau prédéterminé d'IP-10 ou si le niveau d'IL-1β ou d'IL-1α dans l'échantillon est supérieur au niveau prédéterminé d'IL-1β ou d'IL-1a, alors faire un diagnostic non spécifique selon lequel la patiente présente une vaginose bactérienne asymptomatique et/ou une infection sexuellement transmissible asymptomatique.

2. Procédé selon la revendication 1, qui comporte l'étape consistant à renvoyer la patiente pour un test supplémentaire afin de déterminer la pathologie ou l'infection particulière.

3. Procédé selon l'une des revendications 1 ou 2, dans lequel le diagnostic est fait en l'absence de symptômes cliniques d'inflammation dans le tractus génital, de vaginose bactérienne ou d'une infection sexuellement transmissible.

4. Procédé selon l'une quelconque des revendications 1 à 3, dans lequel l'échantillon comprend des sécrétions vaginales qui ont été obtenues de la patiente.

5. Procédé selon la revendication 4, dans lequel l'échantillon provient d'un lavage cervicovaginal, d'un écouvillonnage vulvovaginal, d'un écouvillonnage de paroi latérale ou d'une cape cervicale de la patiente.

6. Procédé selon l'une quelconque des revendications 1 à 5, dans lequel l'étape (a) est effectuée sur un échantillon non stimulé.

7. Procédé selon l'une quelconque des revendications 1 à 6, dans lequel les taux d'une ou plusieurs cytokines supplémentaires choisies dans le groupe constitué de TNF-α, TNF-β, MDC, IL-7, IFN-γ et GRO sont mesurés et comparés à des niveaux prédéterminés afin de faire le diagnostic.

8. Procédé selon l'une quelconque des revendications 1 à 7, dans lequel la patiente est séropositive.

9. Procédé selon l'une quelconque des revendications 1 à 7, dans lequel la patiente est séronégative.
